(19) Europäisches Patentamt / European Patent Office / Office européen des brevets

(11) **EP 4 015 653 B1**

(12) **EUROPEAN PATENT SPECIFICATION**

(45) Date of publication and mention
of the grant of the patent:
**11.06.2025  Bulletin 2025/24**

(21) Application number: **21215086.6**

(22) Date of filing: **16.12.2021**

(51) International Patent Classification (IPC):
***C12Q 1/6886*** (2018.01)

(52) Cooperative Patent Classification (CPC):
**C12Q 1/6886;** C12Q 2600/118; C12Q 2600/158;
C12Q 2600/178

(54) **USE OF MIRNAS AS DIAGNOSTIC AND PROGNOSTIC BIOMARKERS OF ORAL SQUAMOUS CELL CARCINOMA**

VERWENDUNG VON MIRNAS ALS DIAGNOSTISCHE UND PROGNOSTISCHE BIOMARKER EINES ORALEN SCHUPPENZELLKARZINOMS

UTILISATION DE MIARN EN TANT QUE BIOMARQUEURS DE DIAGNOSTIC ET DE PRONOSTIC D'UN CARCINOME MALPIGHIEN DE LA CAVITÉ BUCCALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **16.12.2020  IT 202000031061**

(43) Date of publication of application:
**22.06.2022  Bulletin 2022/25**

(73) Proprietors:
• **Universita' degli studi di Brescia
25121 Brescia (IT)**
• **Universita' Degli Studi Di Padova
35122 Padova (IT)**

(72) Inventors:
• **ROMANI, Chiara
26100, Cremona (IT)**
• **BOSSI, Paolo
20017, Samarate-Varese (IT)**
• **BIGNOTTI, Eliana
25024, Leno-Brescia (IT)**
• **PADERNO, Alberto
25123, Brescia (IT)**
• **ROMUALDI, Chiara
35010, Vigonza-Padova (IT)**
• **NICOLAI, Piero
46049, Volta Mantovana-Mantova (IT)**
• **SALVIATO, Elisa
30031, Dolo-Venezia (IT)**

(74) Representative: **Zaccaro, Elisabetta et al
Notarbartolo & Gervasi S.p.A.
Viale Achille Papa, 30
20149 Milano (IT)**

(56) References cited:
**WO-A1-2017/079571    JP-A- 2020 068 673**

• **CHANG YI-AN ET AL: "A Three-MicroRNA Signature as a Potential Biomarker for the Early Detection of Oral Cancer", INTERNATIONAL JOURNAL OF MOLECULAR SCIENCES, vol. 19, no. 3, 7 March 2018 (2018-03-07), pages 758, XP055831758, DOI: 10.3390/ijms19030758**
• **ROY ROSHNI ET AL: "MicroRNA and target gene expression based clustering of oral cancer, precancer and normal tissues", GENE, ELSEVIER AMSTERDAM, NL, vol. 593, no. 1, 8 August 2016 (2016-08-08), pages 58 - 63, XP029735539, ISSN: 0378-1119, DOI: 10.1016/ J.GENE.2016.08.011**
• **WONG NATHAN ET AL: "signatures derived from The Cancer Genome Atlas for head and neck squamous cell carcinomas", CANCER MEDICINE, vol. 5, no. 7, 1 July 2016 (2016-07-01), GB, pages 1619 - 1628, XP055831858, ISSN: 2045-7634, DOI: 10.1002/cam4.718**
• **LI SIMIN ET AL: "Complex integrated analysis of lncRNAs-miRNAs-mRNAs in oral squamous cell carcinoma", ORAL ONCOLOGY, vol. 73, 1 October 2017 (2017-10-01), GB, pages 1 - 9, XP055831873, ISSN: 1368-8375, DOI: 10.1016/ j.oraloncology.2017.07.026**

EP 4 015 653 B1

• PEDERSEN NICKLAS JUEL ET AL: "MicroRNA-based classifiers for diagnosis of oral cavity squamous cell carcinoma in tissue and plasma", ORAL ONCOLOGY, vol. 83, 1 August 2018 (2018-08-01), GB, pages 46 - 52, XP055831875, ISSN: 1368-8375, DOI: 10.1016/j.oraloncology.2018.05.020

• CERVIGNE NILVA K ET AL: "Identification of a microRNA signature associated with progression of leukoplakia to oral carcinoma", HUMAN MOLECULAR GENETICS,, vol. 18, no. 24, 15 December 2009 (2009-12-15), pages 4818 - 4829, XP002598390, ISSN: 0964-6906, [retrieved on 20090923], DOI: 10.1093/HMG/DDP446

• MOMEN-HERAVI F ET AL: "Genomewide Study of Salivary MicroRNAs for Detection of Oral Cancer", JOURNAL OF DENTAL RESEARCH, INTERNATIONAL ASSOCIATION FOR DENTAL RESEARCH, US, vol. 93, no. 7, Suppl. 1, 1 July 2014 (2014-07-01), pages 86S - 93S, XP002750649, ISSN: 0022-0345, [retrieved on 20140409], DOI: 10.1177/0022034514531018

Remarks:
The complete document including Reference Table(s) and the Sequence Listing(s) can be downloaded from the EPO website

# EP 4 015 653 B1

**Description**

FIELD OF THE INVENTION

The present invention concerns the field of biomarkers suitable for the diagnosis and prognosis of cancer, and in particular relates to the use of miRNAs as a diagnostic and prognostic markers for oral carcinoma.

The present invention further describes a method for diagnosing or for making a prognosis of an oral carcinoma, comprising at least the step of determining the level of miRNA expression in a sample.

STATE OF THE ART

In the last decades, the incidence of oral squamous cell carcinoma (OSCC) has progressively been increasing, with approximately 355,000 new cases in 2018. At the same time, no major improvement in survival outcomes has been observed. Conventional treatment for locally advanced disease comprises wide surgical resection of the tumor, neck dissection and adjuvant (chemo)-radiotherapy according to pathological staging and risk factors. In view of the crucial role of this anatomic site in many physiological conditions, surgery is carefully tailored in order to find the optimal balance between functional and oncologic outcomes. In this regard, the introduction of compartmental approaches, which can be modulated according to tumor extension, can be considered a significant advancement. However, preoperative evaluation only relies on clinical and radiologic data, without considering tumor biology and microenvironment. Indeed, different studies demonstrated that tumors with similar TNM staging may present wide differences in biologic features having an impact on prognosis (i.e., worst pattern of invasion, tumor budding, tumor-stroma ratio and gene-expression signatures). However, the small size of preoperative tumor biopsies does not allow to obtain a representative profile of the different neoplastic cell subpopulations, their pattern of invasion and related risk factors. In fact, distinct areas of OSCC may express a diverse array of features, thus preventing a comprehensive tumor characterization through an incisional biopsy alone.

miRNAs are small non-coding RNA molecules, which show remarkable stability in biologic fluids and altered expression in various cancers, both promising characteristics for the diagnostic and prognostic definition of tumors by means of a liquid biopsy. JP 2020068673 A and Chang Yi-An et al, 2018 (INTERNATIONAL JOURNAL OF MOLECULAR SCIENCES, vol. 19, no. 3, 7 March 2018) show for instance that expression level of microRNAs, including miR-423-5p, in body fluid samples can be used for the diagnosis and prognosis of oral cancer.

Additional tools are strongly needed in OSCC. The need and importance is increasingly felt for the development of a new tool which allows to arrive at an early diagnosis without an invasive sampling process, and which allows to make a prognosis of disease outcome.

SUMMARY OF THE INVENTION

The present invention concerns the use of a combination of target miRNAs:miR-423-5p, miR-106b-5p and miR-193b-3p, as diagnostic markers for diagnosing an oral carcinoma.

A further aspect of the present invention is a method for diagnosing or for making a prognosis of an oral carcinoma, comprising
the steps of:

- determining the level of expression of the miRNA miR-423-5p in a sample; and

- comparing said levels of expression of the miRNAs miR-423-5p, miR-106b-5p and miR-193b-3p in a sample.

The critical issue underlying the present invention lies in making available a biomarker capable of identifying an oral carcinoma with a non-invasive procedure, in order to permit an easier and earlier diagnosis which would allow to save lives.

As will be further described in the detailed description of the invention, the problem is resolved by the present finding by the use of the combination of 3 miRNAs, as identified in the attached claims.

BRIEF DESCRIPTION OF THE DRAWINGS

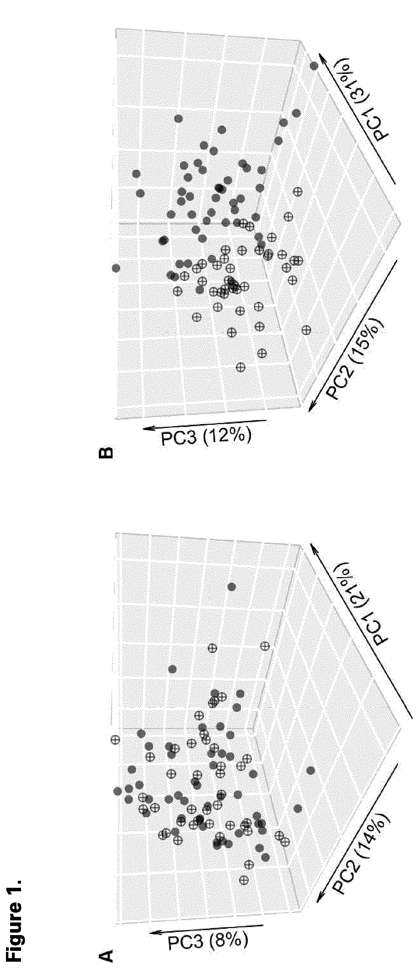

The characteristics and advantages of the present invention will be apparent from the detailed description reported below, from the Examples given for illustrative purposes, and from the annexed Figures 1-5, wherein:

**Figure 1: Principal component analysis of microarray expression profiles.**

Scatter plot of the first three principal components using **(A)** the entire collection of miRNAs profiles (n=826) and **(B)** only the differentially expressed ones (n=25), in OSCC patients (grey dots) and healthy donors (cross dots). The percentage of explained variance for each principal component is reported in brackets.

**Figure 2. Boxplots of the three candidate biomarkers evaluated by RT-qPCR in two cohorts.**

Comparison of $2^{-\Delta Cq}$ values in OSCC patients and healthy controls in training (light grey dots), validation (grey dots) and merged cohorts, (one-tails *t* test, ***$p \leq 0.001$, **$p \leq 0.01$, *$p \leq 0.05$, ^$p \leq 0.1$).

**Figure 3. ROC curves and AUC values of the three candidate miRNA biomarkers.**

Diagnostic performances of the three candidate miRNAs ($2^{-\Delta Cq}$) and their integration employing a logistic model in the training **(A)** and the merged cohorts **(B)**. For the latter, the average of twenty repeated 5-fold cross-validated roc curves is depicted.

**Figure 4. Kaplan-Meier curves and Coxph survival analysis showing the prognostic potential of miR-423-5p.**

Comparison of the prognostic value of the *three-groups* schema (upper panels) that account only for the number of positive lymph nodes (NL) and the *four-groups* model (bottom panels) that integrates miR-423-5p profile in intermediate risk group stratification, in the training (left panels) and merged coorts (right panels).

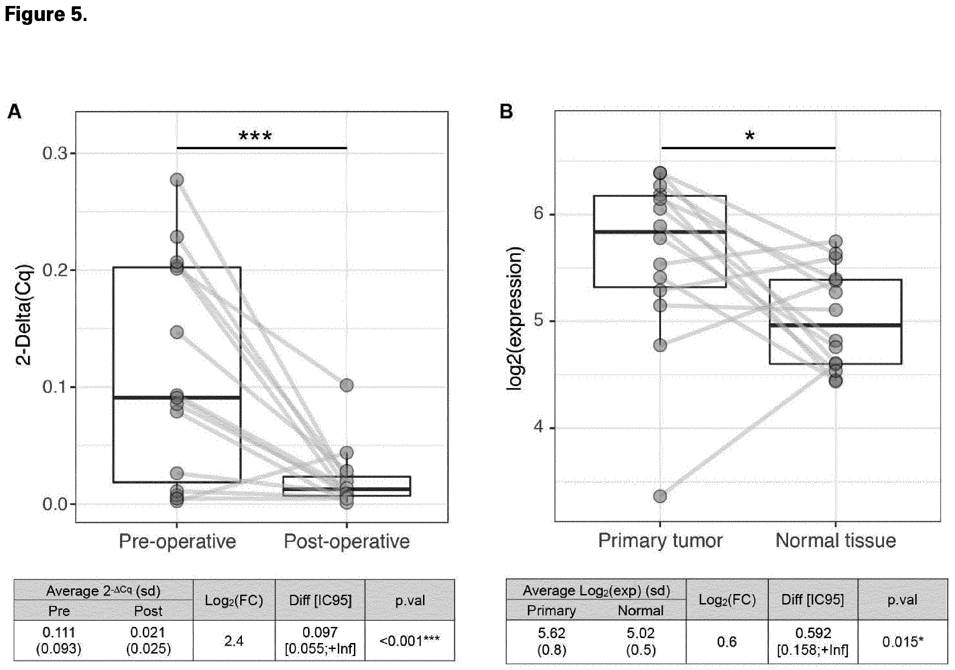

**Figure 5. Evaluation of mir-423-5p tumor specificity.**

Comparison $2^{-\Delta Cq}$ values in **(A)** paired pre- and post-operative samples (n=15) and **(B)** log2 expressions in tumors and matched normal tissues from TCGA collection (n=14), in OSCC patients and healthy controls, (one-tail t test, ***$p \leq 0.001$, *$p \leq 0.05$).

## DETAILED DESCRIPTION OF THE INVENTION

The present invention concerns the use of a combination of target miRNAs for diagnosing an oral carcinoma, said combination of target miRNAs consisting of miRNAs miR-423-5p, miR-106b-5p and miR-193b-3p. Moreover, miR-423-5p shows potential as prognostic biomarker for OSCC.

As used herein, the term "miR-423-5p" refers to a miRNA having a miRBase database accession number MI0001445, having the following sequence:

AUAAAGGAAGUUAGGCUGAGGGGCAGAGAGCGAGACUUUUCUAUUUUCCAAAA

GCUCGGUCUGAGGCCCCUCAGUCUUGCUUCCUAACCCGCGC (SEQ ID NO:1).

Similarly, the term "miR-106b-5p" refers to a miRNA having a miRBase database accession number MI0000734, having the following sequence:

CCUGCCGGGGCUAAAGUGCUGACAGUGCAGAUAGUGGUCCUCUCCGUGCUAC

CGCACUGUGGGUACUUGCUGCUCCAGCAGG (SEQ ID NO:2).

Finally, the term "miR-193b-3p" refers to a miRNA having a miRBase database accession number MI0003137, having the following sequence:

GUGGUCUCAGAAUCGGGGGUUUUGAGGGCGAGAUGAGUUUAUGUUUUAUCCAA

CUGGCCCUCAAAGUCCCGCUUUUGGGGUCAU (SEQ ID NO:3).

The miRNAs: miR-423-5p, miR-106b-5p and miR-193b-3p are target miRNAs.

The reference miRNAs are:

- "miR-16-5p" which has the miRBase database accession number: MIMAT0000069 and the sequence: UAGCAGCACGUAAAUAUUGGCG (SEQ ID NO:4);
- "miR-484" which has the miRBase database accession number: MI0002468 and the sequence:

AGCCUCGUCAGGCUCAGUCCCCUCCCGAUAAACCCCUAAAUAGGGACUUUCCC

GGGGGGUGACCCUGGCUUUUUUGGCG (SEQ ID NO:5);

and

- "miR-191-5p" which has the miRBase database accession number: MIMAT0000440 and the sequence: CAACGGAAUCCCAAAAGCAGCUG (SEQ ID NO:6).

The oral carcinoma that is diagnosed with a combination of target miRNAs consisting of miRNAs miR-423-5p, miR-106b-5p and miR-193b-3, and the oral carcinoma for which a prognosis is made with the miR-423-5p miRNA, can be an oral squamous cell carcinoma.

In a further preferred aspect, the combination of miRNAs consists of miR-423-5p, miR-106b-5p and miR-193b, used for diagnosing or the miR-423-5p used for making a prognosis of an oral carcinoma, is a salivary miRNA.

Salivary miRNAs have emerged as excellent non-invasive cancer biomarker candidates, but their association with OSCC prognosis has not been investigated yet. The present invention analyses global salivary miRNA expression profile in OSCC patients and healthy controls, with the aim of defining its diagnostic and prognostic potential.

In the conceptual framework of liquid biopsy, saliva has the potential to provide an overall view of OSCC tumor heterogeneity, thanks to its capability of collecting molecules originating from different cellular populations and neoplasm sites. Moreover, salivary analysis may offer significant advantages, since it requires a non-invasive procedure and can be performed in the preoperative period, without altering radiologic imaging quality, nor adding discomfort to the patients. Previous studies showed that saliva contains high levels of miRNAs that are protected by enzymatic degradation and that their concentration is higher than in plasma, which might designate a higher sensitivity in detecting changes of expression.

Under a second aspect the present invention relates to a method for diagnosing or making a prognosis of an oral carcinoma, comprising the steps of:

- determining the level of expression of the miRNA miR-423-5p in a sample; and

- comparing said levels of expression of the miRNAs miR-423-5p, miR-106b-5p and miR-193b-3p in a sample.

By the term "diagnosing" it is intended to identify the nature of the medical condition, while by "making a prognosis" it is meant that an opinion of the likely course of a medical condition is made.

In a preferred aspect, the method for diagnosing or making a prognosis of an oral carcinoma according to the invention comprises the further step of comparing said levels of expression of the miRNAs miR-423-5p, miR-106b-5p and miR-193b-3p (target miRNAs) in a sample to the level of expression of the reference miRNAs miR-16-5p, miR-484 and miR-191-5p.

In a still preferred aspect, in the method for diagnosing an oral carcinoma according to the invention said method comprises at least the steps of:

(a) determining the expression level (Cq) of the *target* miRNAs (miR-423-5p, miR-106b-5p and miR-193b-3p) in a sample;
(b) determining the expression (Cq) level of the *reference* miRNAs (miR-16-5p, miR-484 and miR-191-5p) in a sample;
(c) normalizing the expression level of the target miRNAs to the expression of the reference miRNAs (ΔCq), *i.e.,* subtract to the measured expression level of each target the arithmetic mean expression of the reference miRNAs;
(d) calculating the *diagnostic score* as linear combinations of normalized expression levels of target miRNAs. Namely:

$$-0.52293 -0.35877\times\Delta Cq^{miR\text{-}106b\text{-}5p} +0.11394\times\Delta Cq^{miR\text{-}193b\text{-}3p} +1.00474\times\Delta Cq^{miR\text{-}423\text{-}5p} ;$$

(e) comparing the diagnostic score with a predetermined cutoff value chosen to exclude oral carcinoma, wherein a diagnostic score above the cutoff value of -0.48426 is indicative of oral carcinoma.

In the present invention, making a prognosis of the outcome of an oral carcinoma means the estimate of the likely course of the disease.

In a still preferred aspect, in the method for making a prognosis of the outcome of an oral carcinoma - for patient with intermediate risk of relapse (*i.e.,* with at least one lymph node, but lower than five) - according to the invention said method comprises at least the steps of:

(a) determining the expression level (Cq) of the miRNA miR-423-5p in a sample;
(b) determining the expression level of the *reference* miRNAs (miR-16-5p, miR-484, miR-191-5p) in a sample;
(c) normalizing the expression level of miR-423-5p to the expression of the reference miRNAs (ΔCq), *i.e.,* subtract to the measured expression level of miRNA-423-5p the arithmetic mean expression of the reference miRNAs;

(d) comparing the normalized expression level of miRNA-423-5p with a predetermined cutoff value to predict risk of relapse for oral carcinoma, wherein a normalized expression level above the cutoff value of 0.49 is indicative of an increased risk of oral carcinoma relapse.

In a further aspect, the invention relates to a method for diagnosing or for making a prognosis of an oral carcinoma by determining the level of expression of the target miRNAs miR-423-5p, miR-106b-5p and miR-193b-3p and the reference miRNAs miR-16-5p, miR-484 and miR-191-5p in a sample, wherein said sample is a liquid biopsy. Preferably said sample is a saliva sample.

In a more preferred aspect, the invention relates to a method for diagnosing or for making a prognosis of an oral carcinoma by determining the level of expression of the miRNA miR-423-5p in a sample, wherein said level of expression determination is carried out by real-time PCR.

In order to validate the method of the invention, saliva was collected from patients with newly diagnosed untreated primary OSCC and healthy controls. Global profiling of salivary miRNAs was carried out through a microarray approach, while signature validation was performed by quantitative real-time PCR (RT-qPCR). A stringent statistical approach for microarray and RT-qPCR data normalization was applied. The diagnostic performance of miRNAs and their correlation with OSCC prognosis were comprehensively analyzed.

In total, as will be seen in the Examples section, 25 miRNAs emerged as differentially expressed between OSCC patients and healthy controls and, among them, seven were significantly associated with disease-free survival (DFS). miR-106b-5p, miR-423-5p and miR-193b-3p were expressed at high levels in saliva of OSCC patients and their combination displays the best diagnostic performance (ROC - AUC = 0.98). Moreover, high expression of miR-423-5p was an independent predictor of poor DFS, when included in multivariate survival analysis with the number of positive lymph nodes - the only significant clinical prognosticator. Finally, a significant decrease in miR-423-5p expression was observed in matched post-operative saliva samples, suggesting its potential cancer-specific origin.

In conclusion, salivary miRNAs identified in the cohort of patients show a surprising and unexpected accuracy in OSCC detection and to effectively stratify patients according to their likelihood of relapse. These results, are particularly promising for screening/follow-up of high-risk populations and useful for preoperative prognostic assessment.

As will be evident from the Examples, first, preoperative saliva samples from OSCC patients and healthy controls were employed to investigate whether expression levels of specific miRNAs could be used as diagnostic biomarkers and, subsequently, their impact on prognosis was explored. This was performed using a standardized stepwise approach, through comprehensive evaluation of global miRNA expression in saliva, validation of candidate miRNAs, normalization by means of carefully selected stable endogenous controls and analysis of their diagnostic and prognostic potentials. The results confirmed the promising value of three salivary miRNAs, miR-106b-5p, miR-423-5p and miR-193b-3p, which showed high and specific levels of expression in OSCC patients, with a remarkable diagnostic performance, potentially allowing detection of OSCC through salivary analysis alone. Importantly, an additional cohort of OSCC patients and healthy controls in which the diagnostic value of miR-106b-5p, miR-423-5p and miR-193b-3p have been evaluated by RT-qPCR and confirmed was included in the study.

In countries with relatively low incidence of OSCC, a highly effective diagnostic test could be regarded as a tool for secondary prevention (i.e., screening programs) only in at-risk populations (i.e., heavy smokers, alcohol abusers and patients with lesions at risk of malignant progression and/or an history of previous head and neck cancers). Moreover, patients previously treated for OSCC represent a particularly high-risk cohort that could significantly benefit from early diagnosis of recurrence. In fact, relapse frequently presents as locally advanced disease regardless of the follow-up policy, thus increasing the invasiveness of salvage treatment and reducing its effectiveness. In this view, this study could represent a first step towards validation of saliva-based screening methods that could help improving diagnosis in the initial stages of cancer recurrence. Among clinical risk factors in OSCC, we confirmed in our series the critical prognostic role of the number of positive lymph nodes. This variable was considered in conjunction with salivary miRNA expression, identifying miR-423-5p as a significant prognosticator in multivariate analysis. Interestingly, miR-423-5p showed a complex and clinically-relevant interrelation with nodal status, allowing risk stratification of patients with 1-4 positive LNs, a subgroup comprising the vast majority of node-positive patients in OSCC. Patients with 1-4 positive LNs and low salivary miR-423-5p expression were classified as low risk, with a disease-free survival (DFS) comparable to that of node-negative patients. On the other hand, those with 1-4 positive LNs and a high salivary miR-423-5p expression were classified as high risk and presented a significantly lower DFS (comparable with that of patients with five or more positive LNs). Notably, those findings have been validated in a second cohort of OSCC patients, in which miR-423-5p expression was also confirmed to be useful in classifying patients in the intermediate-risk class.

This result is noteworthy considering that recent clinical reports highlighted the prominent independent prognostic role of positive LN number, surpassing even extranodal extension, and being proposed as a novel method for N stratification in the TNM system (Mattavelli D, et al. Prognostic nomograms in oral squamous cell carcinoma: the negative impact of low neutrophil to lymphocyte ratio. Front Oncol 2019;9:339). In this context, salivary miR-423-5p may allow a further risk stratification that could lead to better treatment personalization, especially with regard to adjuvant therapy. To date, according to the National Comprehensive Cancer Network guidelines, postoperative radiotherapy is recommended in patients with minor adverse features (i.e., pT3 or pT4 primary, N2 or N3 nodal disease, nodal disease in levels IV or V, perineural invasion, vascular embolism, and lymphatic invasion); conversely, chemoradiotherapy is generally suggested only in those presenting major risk factors (i.e., extranodal extension and positive margins). However, these criteria are only based on the results of two randomized clinical trials published in 2004, and further refinements may significantly improve indications for adjuvant treatment. In this regard, a retrospective observational cohort study using the National Cancer Database showed chemoradiotherapy having a survival benefit in comparison to radiation among patients with node-positive, resected, locally advanced head and neck squamous cell cancers, without pathologic major risk factors. It may be hypothesized that miR-423-5p may better define patient selection, thus improving survival and more precisely profiling adjuvant treatment.

Currently, the source of salivary miRNAs in cancer patients still remains undetermined. The present investigation demonstrates that the diagnostic and prognostic OSCC miRNAs of the present invention have a tumor-associated origin, being significantly reduced after surgical resection and overexpressed in OSCC compared to normal tissues.

Various embodiments and aspects of the present invention as delineated hereinabove and as claimed in the claims section below find experimental support in the following examples.

EXAMPLES

Reference is now made to the following examples, which together with the above descriptions illustrate some embodiments of the invention.

**Example 1.**

Discovery of potential salivary miRNA biomarkers by microarray

A total of 147 subjects were enrolled in the study, including 58 healthy individuals and 89 OSCC patients, divided into a training set and a test set according to the study design. Clinicopathological characteristics are summarized in Table 1.

Table 1. Clinicopathological features of OSCC patients and healthy donors.

| Characteristics | Class | | Training OSCC (n=61), n (%) | Training Healthy (n=44), n (%) | Validation OSCC (n=28), n (%) | Validation Healthy (n=14), n (%) |
|---|---|---|---|---|---|---|
| Age (years) | Mean [(range]) | | 66.7 [(30-90]) | 50.72 [(22-92]) | 64.75 [24-91] | 75.57 [71-91] |
| Gender | Female | | 18 (30) | 16 (36) | 9 (32) | 4 (29) |
|  | Male | | 43 (70) | 28 (64) | 19 (68) | 10 (71) |
| Smoke | No | | 25 (41) | 23 (52) | 14 (50) | 4 (30) |
|  | Yes | | 36 (59) | 21 (48) | 13 (46) | 5 (35) |
|  | NA | | - | - | 1 (4) | 5 (35) |
| Alcohol | No | | 29 (47) | 19 (43) | 22 (78) | 6 (43) |
|  | Yes | | 32 (53) | 25 (57) | 5 (18) | 6 (43) |
|  | NA | | - | - | 1 (4) | 2 (14) |
| N. of positive lymph nodes | Mean (range) | | 1.6 [0;13] | - | 1.88 [0;7] | - |
| Stage (TNM) | pT | pT1 | 8 (13) | - | 6 (21) | - |

(continued)

| Characteristics | Class | | Training OSCC (n=61), n (%) | Training Healthy (n=44), n (%) | Validation OSCC (n=28), n (%) | Validation Healthy (n=14), n (%) |
|---|---|---|---|---|---|---|
| | | pT2 | 15 (25) | | 6 (21) | |
| | | pT3 | 25 (41) | | 8 (29) | |
| | | pT4 | 9 (15) | | 8 (29) | |
| | | NA | 4 (6) | | - | |
| | pN | pN0 | 36 (59) | - | 11 (39) | - |
| | | pN1 | 6 (10) | | 2 (7) | |
| | | pN2 | 5 (8) | | 8 (29) | |
| | | pN3 | 10 (17) | | 7 (25) | |
| | | NA | 4 (6) | | - | |
| Extranodal Extension | No | | 10 (47.6) | - | 9 (53) | - |
| | Yes | | 11 (52.4) | | 8 (47) | |
| Tumor site | Non-tongue | Floor of mouth | 8 (13.1) | - | 2 (7.1) | - |
| | | Alveolar crest | 14 (23) | | 6 (21.5) | |
| | | Hard palate | 4 (6.6) | | 2 (7.1) | |
| | | Cheek | 11 (18) | | 4 (14.3) | |
| | Tongue | Tongu e | 24 (39.3) | - | 14 (50) | - |
| Tumor grade | G1 | | 14 (23.0) | - | 5 (17.9) | - |
| | G2 | | 34 (55.7) | | 15 (53.6) | |
| | G3 | | 12 (19.7) | | 8 (28.6) | |
| | NA | | 1 (1.6) | | | |
| Perineural invasion | No | | 31 (50.8) | - | 7 (25) | - |
| | Yes | | 30 (49.2) | | 21 (75) | |
| Endovascular infiltration | No | | 48 (78.7) | - | 14 (50) | - |
| | Yes | | 13 (21.3) | | 14 (50) | |
| Bone infiltration | No | | 50 (82.0) | - | 19 (67.9) | - |
| | Yes | | 11 (18.0) | | 9 (32.1) | |
| Complementary therapy | No | | 26 (42.6) | | 11 (39.3) | |
| | Yes | | 34 (55.7) | | 15 (53.6) | |
| | NA | | 1 (1.6) | | 2 (7.1) | |

In the discovery stage, we performed global miRNA expression profile on preoperative saliva from 50 patients with primary OSCC and 42 healthy controls (training set), with the aim of identifying miRNAs both differentially expressed between OSCC patients and healthy subjects and characterized by the potential of predicting prognosis, in terms of DFS in OSCC patients.

Salivary raw microarray data comprised 1361 miRNAs. Among them, 826 miRNAs fulfilled the filtering criteria described in the method section and resulted as detected in saliva samples. In total, 25 out of 826 miRNAs (3%) were identified to be differentially expressed (adjusted $p \leq 0.05$) between saliva of OSCC patients and healthy controls (Table 2), indicating a distinct salivary miRNA profile in OSCC patients (Figure 1).

Twenty out of 25 miRNAs (80%) were significantly upregulated in saliva samples of OSCC patients compared to controls, whereas five miRNAs (20%) were downregulated. To further discriminate potential prognosticator candidates among the differentially expressed miRNAs, we evaluated survival association, considering DFS as the endpoint. In univariate survival analysis, seven out of 25 miRNAs were significantly associated with DFS with a relaxed threshold of 0.1 (Table 2).

Table 2. Panel of 25 differentially expressed miRNAs (Ebayes, p ≤ 0.05) along with results of Coxph analysis for disease-free survival based on microarray log2 expression. MiRNAs that satisfied criteria for subsequent validation are highlighted (green). Number of samples: 92 (OSCC=50; Healthy=42).

| miRNA | Average Log2(exp) | Diagnostic relevance (OSCC + healthy); Ebayes | | Prognostic relevance (OSCC); DFS, Coxph | |
|---|---|---|---|---|---|
| | | logFC (OSCC vs healthy) | Adjusted p-value | HR [95% CI] | p-value |
| miR-30c-5p | 5.0 | -0.632 | <0.001*** | 0.7 [0.3–1.9] | 0.473 |
| miR-654-5p | 5.6 | 0.970 | <0.001*** | 1.2 [0.8–1.8] | 0.424 |

| | | | | | |
|---|---|---|---|---|---|
| miR-106b-5p | 7.4 | -0.705 | 0.001*** | 2.0 [1.0–3.8] | 0.038* |
| miR-3680-3p | 4.3 | 1.156 | 0.002** | 0.7 [0.5–0.9] | 0.011* |
| miR-320b | 6.9 | 0.553 | 0.002** | 1.7 [0.8–3.5] | 0.133 |
| miR-224-5p | 4.9 | 0.988 | 0.002** | 1.3 [0.8–1.9] | 0.275 |
| hsv2-miR-H9-5p | 8.1 | -1.846 | 0.002** | 0.8 [0.7–1.0] | 0.118 |
| miR-320a | 6.9 | 0.545 | 0.008** | 0.6 [0.3–1.2] | 0.120 |
| miR-1290 | 12.1 | 1.091 | 0.008** | 1.1 [0.8–1.7] | 0.512 |
| miR-1275 | 7.9 | 0.797 | 0.010** | 0.9 [0.5–1.4] | 0.536 |
| miR-320c | 8.9 | 0.736 | 0.010** | 1.2 [0.8–1.8] | 0.412 |
| miR-30b-5p | 5.7 | -0.597 | 0.010** | 0.8 [0.41.5] | 0.473 |
| miR-130a-3p | 5.6 | 0.830 | 0.012* | 1.5 [1.0–2.1] | 0.039* |
| miR-3190-3p | 4.3 | 0.888 | 0.014* | 0.5 [0.3–0.7] | <0.001*** |
| miR-3692-5p | 4.6 | 0.729 | 0.014* | 1.1 [0.7–1.5] | 0.789 |
| miR-26a-5p | 7.2 | -0.554 | 0.014* | 0.9 [0.4–1.9] | 0.709 |
| kshv-miR-K12-7-5p | 4.5 | 0.624 | 0.019* | 1.0 [0.7–1.6] | 0.833 |
| miR-320e | 7.0 | 0.483 | 0.023* | 1.8 [1.0–3.4] | 0.065^ |
| miR-887-3p | 4.2 | 0.519 | 0.023* | 1.1 [0.7–1.9] | 0.704 |
| hsv1-miR-H16 | 5.1 | 0.661 | 0.024* | 1.0 [0.6–1.7] | 0.894 |
| miR-320d | 7.5 | 0.469 | 0.024* | 1.4 [0.7–2.7] | 0.341 |
| miR-423-5p | 6.4 | 0.425 | 0.026* | 2.9 [1.1–7.9] | 0.033* |
| miR-193b-3p | 6.1 | 0.601 | 0.042* | 1.9 [1.2–3.2] | 0.007** |
| miR-205-5p | 9.3 | 0.803 | 0.043* | 1.3 [0.9–1.9] | 0.168 |
| miR-1246 | 14.0 | 0.801 | 0.046* | 1.2 [0.8–1.7] | 0.451 |

^≤0.1 *≤0.05 **≤0.01 ***≤0.001.

**Example 2.**

Validation of candidate salivary miRNAs by RT-qPCR

Following microarray data analysis, a set of potential miRNAs was chosen for validation by RT-qPCR, based on the following criteria: i) high expression level (log2 average expression ≥6); ii) commercially available and experimentally validated locked-nucleic acid PCR primer set; and iii) statistical significance in both the comparison between saliva of OSCC patients and healthy controls (≤0.05) and univariate survival analysis (≤0.1).

Four miRNAs (miR-106b-5p, miR-320e, miR-423-5p and miR-193b-3p) were selected according to the defined criteria, as shown in Table 2. Their expression was evaluated by RT-qPCR in a cohort of 55 OSCC patients and 39 healthy controls from the training set, including 48 and 37 microarray-profiled samples, respectively.

A median Cq <30 was considered indicative of a reliable detection regarding circulating miRNA quantification, as reported by other groups (Bianchi F, et al. A serum circulating miRNA diagnostic test to identify asymptomatic high-risk individuals with early stage lung cancer. EMBO Mol Med 2011;3(8):495-503.). MiR-106b-5p, miR-423-5p and miR-193b-3p were confirmed to be expressed at high levels (medianCq = 27.7, $\sigma$Cq = 2.2; medianCq = 28.9, $\sigma$Cq = 1.9; medianCq = 27.9, $\sigma$Cq = 1.9, respectively) in almost all saliva samples (average detection of 99.5%). On the contrary, miR-320e exceeded the reliability threshold and was discarded from further analysis (medianCq=32.5, $\sigma$Cq=1.8).

**Example 3.**

Selection of reference miRNAs for RT-qPCR data normalization

The systematic evaluation of published articles on the analysis of miRNA expression in salivary samples revealed three miRNAs reported to be stably expressed: miR-16-5p, miR-484 and miR-191-5p. The expression of these putative references was evaluated by RT-qPCR and tested for stability in the cohort of 55 OSCC and 39 healthy subjects. Since normalization by multiple references is recommended by MIQE guidelines (Bustin SA, et al. The MIQE guidelines: minimum information for publication of quantitative real-time PCR experiments. Clin Chem 2009;55(4):611-622.), we have also considered all the possible combinations of the three references, as arithmetic mean across each sample.

According to the equivalence tests (Table 3), a normalization factor based on the average expression levels of the three miRNAs was the most significantly equivalent between normal and malignant samples (p=0.004). Interestingly, although miR-191-5p individually showed a borderline equivalence (p =0.105), it is required to enhance the overall stability of the normalization factor. All these findings were confirmed with NormFinder (Table 3) and are in accordance with the literature.

Table 3. Diagnostic and prognostic stability analysis (equivalence test, NormFinder) of three reference candidates and their possible combinations, based on RT-qPCR results. The most overall stable reference is highlighted (yellow). Labels: ①: miR-16-5p, ②: miR-484, ③: miR-191-5p. Number of samples: 94 (OSCC=55; Healthy=39).

| Reference | Average Cq (SD) | | | Diagnostic stability (OSCC + healthy) | | | | Prognostic stability (DFS) (OSCC) | |
| | | | | Equivalence Test[1] | | NormFinder | | Equivalence Test[2] | |
| | NA | OSCC | Healthy | Difference [95% CI] | p-value | Difference (SD) | Stability | log(HR) [95% CI] | p-value |
|---|---|---|---|---|---|---|---|---|---|
| ① | - | 22.7 (1.9) | 22.9 (1.7) | -0.20 [-0.82–0.43] | 0.017* | 0.19 (0.50) | 0.18 | -0.21 [-0.41 to -0.02] | 0.007** |
| ② | 3 | 29.1 (1.8) | 29.4 (1.4) | -0.30 [-0.86–0.25] | 0.020* | 0.32 (0.65) | 0.26 | -0.24 [-0.46 to -0.02] | 0.024* |
| ③ | - | 28.2 (2.0) | 27.8 (1.9) | 0.48 [1.17–0.11] | 0.105^ | 0.51 (0.88) | 0.34 | -0.19 [-0.36 to -0.01] | 0.002** |
| ① + ② | 3 | 25.9 (1.8) | 26.1 (1.5) | -0.24 [-0.82–0.35] | 0.017* | 0.26 (0.40) | 0.20 | -0.23 [-0.44 to -0.02] | 0.017* |
| ① + ③ | - | 25.5 (1.9) | 25.3 (1.7) | 0.14 [-0.49–0.77] | 0.013* | 0.16 (0.26) | 0.13 | -0.22 [-0.41 to -0.02] | 0.008** |
| ② + ③ | 3 | 28.7 (1.8) | 28.6 (1.6) | 0.11 [-0.48–0.77] | 0.007** | 0.10 (0.16) | 0.08 | -0.23 [-0.44 to -0.01] | 0.019* |
| ① + ② + ③ | 3 | 26.7 (1.8) | 26.7 (1.6) | 0.02 [-0.58–0.61] | 0.004** | 0.00 (0.11) | 0.02 | -0.22 [-0.44 to -0.01] | 0.016* |

[1]Magnitude of similarity for difference between groups ($\varepsilon$) =1

[2]Magnitude of similarity for cox regression coefficient ($\gamma$) = 0.5

^≤0.11  *≤0.05 **≤0.01 ***≤0.001

Importantly, the same normalization factor showed to be prognostically stable within the cohort of OSCC patients (p=0.016), since the estimated hazard ratio (HR) for DFS was included in the equivalence margins ($\gamma$=0.5). These results indicate miR-484, miR-16-5p and miR-191-5p as suitable and robust normalizers for relative quantification in both diagnostic and prognostic studies in OSCC.

**Example 4.**

3-miRNA signature as non-invasive diagnostic biomarkers for OSCC patients

The arithmetic mean of miR-484, miR-16-5p and miR-191-5p was used to normalize miR-106b-5p, miR-423-5p and miR-193b-3p raw Cq data (Table 4).

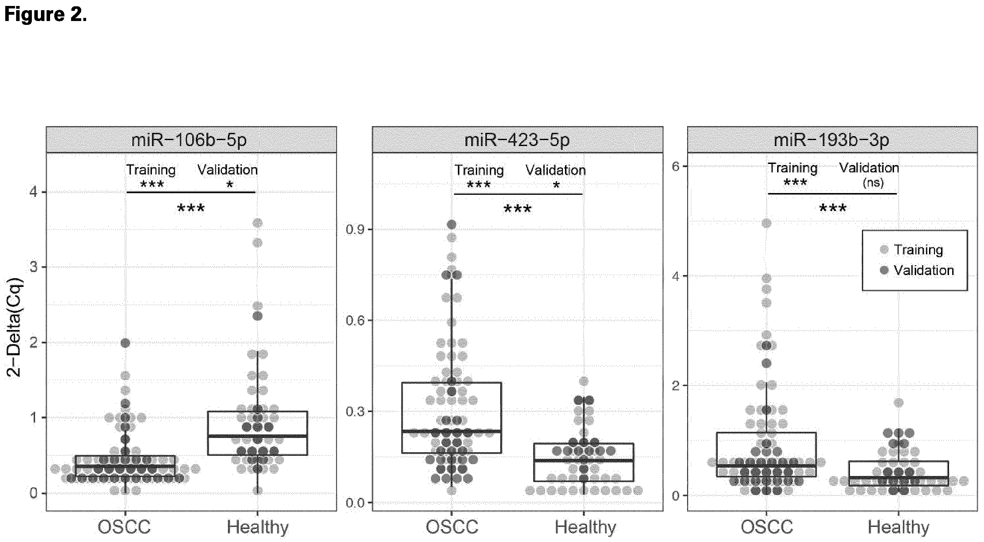

All candidate miRNAs confirmed a significant differential expression in the saliva of OSCC patients compared with healthy controls (Table 4 and Figure 2), in accordance with results and trends of microarray data).

**Table 4.** Differential expressions analysis of the three candidate biomarkers evaluated by RT-qPCR, in the training cohort.
Validated (p ≤ 0.05) diagnostic miRNAs are highlighted (green). Number of samples: 94 (OSCC=55; Healthy=39).

| miRNA | NA | Average $2^{-\Delta Cq}$ | | Log2(FC) | T-test [1] | |
|---|---|---|---|---|---|---|
| | | OSCC | Healthy | OSCC vs healthy | Diff [95% CI] | p-value |
| miR-106b-5p | 3 | 0.453 | 1.003 | -1.15 | -0.550 [-Inf to -0.330] | <0.001*** |
| miR-423-5p | 3 | 0.349 | 0.138 | 1.34 | 0.210 [0.157-Inf] | <0.001*** |
| miR-193b-3p | 4 | 1.085 | 0.382 | 1.51 | 0.740 [0.430–Inf] | <0.001*** |

[1] Student's T-test, one tail.

^≤0.1  *≤0.05 **≤0.01 ***≤0.001

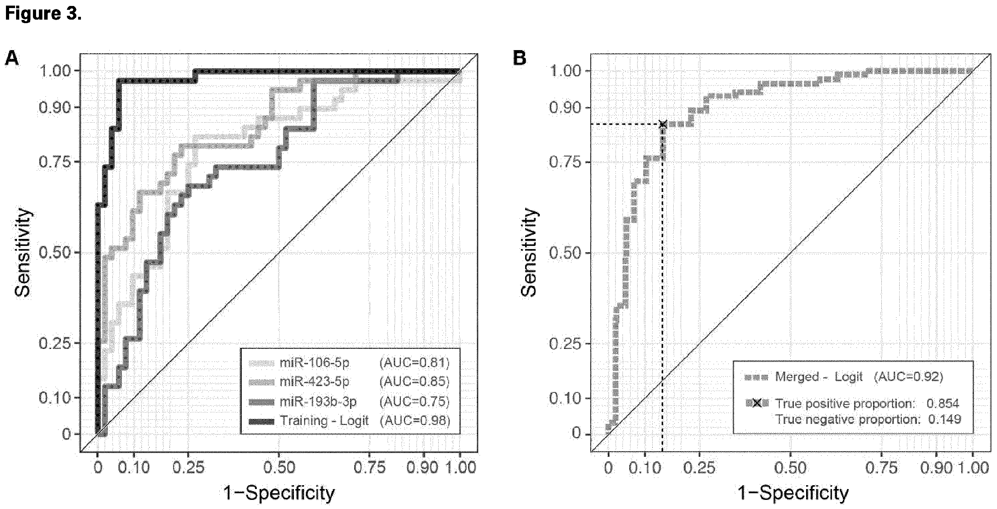

To assess the accuracy of miR-106b-5p, miR-423-5p and miR-193b-3p as diagnostic biomarkers, we estimated ROC curves (Figure 3A), separately on each miRNA expression and on their integration, employing logistic model predictions. The AUC values - based on repeated k-fold cross validation approach - were 0.813 (Se = 0.842; Sp = 0.731), 0.851 (Se = 0.885; Sp = 0.639), 0.748 (Se = 0.750; Sp = 0.639) and 0.98 (Se = 0.974; Sp = 0.942), respectively. The superior performance of the three-miRNA combination highlights its potential role as a diagnostic biomarker for OSCC detection.

**Example 5.**

High miR-423-5p expression is an independent predictor of reduced DFS in OSCC patients

To ascertain whether miR-106b-5p, miR-423-5p and miR-193b-3p exhibited a potential as prognosticators, we conducted a survival analysis using each candidate miRNA normalized expression value (2-ΔCq), either alone in univariate analysis or in combination with the number of positive lymph nodes as covariate in multivariate analysis. As shown in Table 5, the number of positive lymph nodes (LNs) is the only feature associated with DFS in multivariate survival analysis, when considering clinical characteristics.

**Table 5.** Coxph survival analysis of clinicopathological features considered for disease-free survival.

Features selected as covariate for the survival analysis of candidate prognostic miRNAs are highlighted (light blue). Number of samples: 57.

| Feature (classes) | Number in classes | | | DFS – Univariate | | DFS – Multivariate[1] | |
|---|---|---|---|---|---|---|---|
| | 0 | 1 | NA | HR [95% CI] | p-value | HR [95% CI] | p-value |
| Gender (female, male) | 16 | 41 | – | 1.5 [0.6–3.9] | 0.358 | – | – |
| Smoke (no, yes) | 18 | 39 | – | 0.5 [0.2–1.7] | 0.114 | – | – |
| Alcohol (no, yes) | 26 | 31 | – | 1.6 [0.7–3.8] | 0.230 | – | – |
| Tumor site (not-tongue, tongue) | 34 | 23 | – | 0.9 [0.4–2.2] | 0.898 | – | – |

| Feature (classes) | 0 | 1 | NA | HR [95% CI] | p-value | HR [95% CI] | p-value |
|---|---|---|---|---|---|---|---|
| Tumor dimension (<4,=4) | 25 | 32 | – | 2.6 [1.1–6.4] | 0.032* | 1.7 [0.6–4.5] | 0.297 |
| Complementary therapy (no, yes) | 26 | 31 | – | 1.9 [0.8–4.4] | 0.146 | – | – |
| Tumor grade (G1 or G2, G3) | 44 | 11 | 2 | 0.4 [0.1–1.5] | 0.166 | – | – |
| Age | | | – | 1.0 [1.0–1.1] | 0.255 | - | - |
| N. of positive lymph nodes | | | – | 1.3 [1.1–1.4] | <0.001*** | 1.2 [1.1–1.4] | 0.003** |

[1]Multivariate survival model accounted all features resulted significant (*≤0.05) in univariate model.

^≤0.1 *≤0.05 **≤0.01 ***≤0.001

When LNs is added, together with the three candidate miRNAs, to the multivariate survival model (Table 6), miR-423-5p is the only showing independent prognostic potential (p = 0.027). Interestingly, when we replaced the absolute number of positive LNs with the three-group classification proposed by Ho and colleagues (Metastatic lymph node burden and survival in oral cavity cancer. J Clin Oncol 2017;35(31):3601-3609), the role of miR-423-5p became crucial for risk classification in the intermediate/extreme classes (Table 7). The reported evidence suggests the addition to this scheme of a level of stratification based on the miR-423-5p expression for patients with at least one positive LN.

**Table 6.** Coxph survival analysis for candidate prognostic miRNAs evaluated by RT-qPCR. Validated (p ≤ 0.05) prognostic miRNAs are highlighted (green). Number of samples: 55. NL: number of positive lymph nodes.

| miRNA | NA | Average 2^{-ΔCq} [range] | DFS – Univariate | | DFS – Multivariate | | | |
|---|---|---|---|---|---|---|---|---|
| | | | miRNA | | miRNA | | NL | |
| | | | HR [95% CI] | p-value | HR [95% CI] | p-value | HR [95% CI] | p-value |
| miR-106b-5p | 3 | 0.453 [0.004–1.540] | 0.9 [0.2–3.5] | 0.880 | 0.8 [0.2–3.0] | 0.753 | 1.3 [1.1–1.4] | <0.001*** |
| miR-423-5p[a] | 3 | 0.349 [0.052–0.873] | 1.2 [1.0–1.5] | 0.102^ | 1.3 [1.0–1.6] | 0.027* | 1.3 [1.1–1.5] | <0.001*** |
| miR-193b-3p | 3 | 1.085 [0.007–4.959] | 1.5 [0.8–1.6] | 0.448 | 1.1 [0.7–1.7] | 0.559 | 1.3 [1.1–1.4] | <0.001*** |

^≤0.11 *≤0.05 **≤0.01 ***≤0.001

[a] Confidence interval based on an increment of 0.1 unit of 2^{-ΔCq}

**Table 7.** Interactions between miR-423-5p profile and the three classes of positive lymph nodes (NL) in disease-free survival prediction (Coxph). Number of samples: 55 (NA=3).

| Variable | HR [95% CI] | p-value |
|---|---|---|
| NL=0 | Reference group | |
| miR-423-5p[a] | 1.11 [0.85-1.45] | 0.431 |
| (miR-423-5p[a]):(1≤NL<5) | 1.25 [1.00-1.57] | 0.048* |
| (miR-423-5p[a]):(NL≥5) | 1.75 [1.21-2.52] | 0.003** |
| ^≤0.10 *≤0.05 **≤0.01 ***≤0.001 [a]Confidence interval based on an increment of 0.1 unit of 2^{-ΔCq} | | |

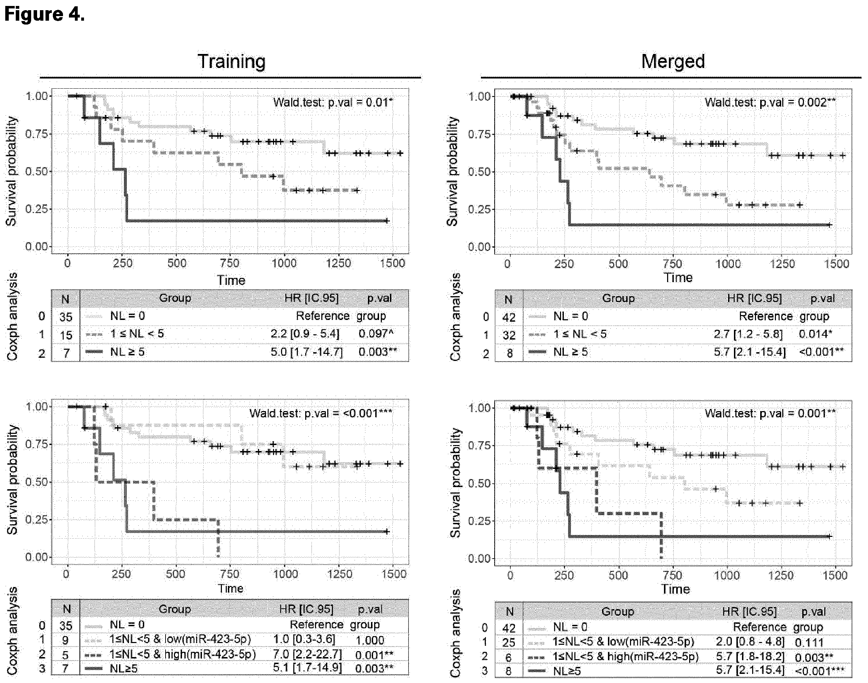

Due to the low sample size of the high-risk group (LN ≥ 5), we evaluated the miRNA integration only in the intermediate one (1 ≤ LN < 5), using a split strategy based on the mean level (cut.off = 0.49). Compared to the LN three-group classification (Figure 4a, p = 0.01), miR-423-5p expression improves the overall ability to predict DFS in OSCC patients (Figure 4b, p < 0.001): patients of the intermediate class with low levels of miR-423-5p exhibit a HR comparable to those with negative LNs; on the contrary, patients of the intermediate class with high levels of miR-423-5p are characterized by reduced DFS, consistent with those with five or more positive LNs.

**Example 6.**

Diagnostic and prognostic performance of salivary miRNAs is confirmed in a second cohort of OSCC and healthy subjects

To ensure the robustness of the proposed biomarkers, we tested their diagnostic and prognostic value in a second cohort of independent individuals (hereinafter, the validation cohort), composed of 28 OSCC patients and 14 healthy subjects (Table 1). Cq values, after removal of batch effects, were normalized using the mean of the reference miRNAs (mean(Cq): OSCC=26.69, Healthy: 26.65 - equivalence p.val=0.016). Due to age biases in the control set (Healthy: [71-91] years; OSCC: [24-91] years), a further correction was required in the comparison between the two groups within this cohort.

The diagnostic performances of the miR-423-5p and miR-106b-5p were substantiated (Figure 2 and Table 8), contrary to miR-193b-3p (p.val = 0.144). However, these results arise from the lower statistical power of the validation set: repeating the comparison on the merged cohorts (Figure 2) all the three biomarkers achieve extremely high significance (p <0.001).

**Table 8.** Differential expression analysis of the three candidate biomarkers evaluated by RT-qPCR, in the validation cohort. Validated (p ≤ 0.05) diagnostic miRNAs are highlighted (green). Number of samples: 42 (OSCC=28; Healthy=14).

| miRNA | NA | Average $2^{-\Delta Cq}$ | | Log2(FC) | T-test [1] | |
|---|---|---|---|---|---|---|
| | | *OSCC* | *Healthy[2]* | OSCC vs healthy | *Diff [95% CI]* | *p-value* |
| miR-106b-5p | 0 | 0.666 | 1.287 | -0.95 | -0.620 [-Inf to -0.188] | 0.011* |
| miR-423-5p | 0 | 0.147 | 0.101 | 0.54 | 0.050 [0.005–Inf] | 0.033* |
| miR-193b-3p | 0 | 0.225 | 0.174 | 0.38 | 0.052 [-0.029–Inf] | 0.144 |

[1] Student's T-test, one tail. [2] Corrected by batch-effect and age.

^≤0.1  *≤0.05 **≤0.01 ***≤0.001

Combining the three-miRNAs signatures (Figure 3B), employing the logistic model together with the repeated k-fold cross-validation approach, we can effectively discriminate OSCC patients and healthy controls also in the extended cohort, with a robust AUC estimate equal to 0.923 and the associated sensitivity and specificity of 0.854 and 0.851, respectively.

By adding new OSCC patients to the DFS analysis (Figure 4, left panels), we duplicated the observations in the intermediate-risk class (from 15 to 32 patients) which became significantly different from the group with no lymph nodes (HR=2.7, p.val = 0.014). Applying the cut-off outlined in the previous paragraph, only one out of 17 new patients fall into the high-risk intermediate class, while all the remaining are in the low-risk intermediate class. Overall, the integration of the miR-423-5p is established as useful in the classification strategy (Wald test: p.val = 0.001), proving the higher similarity of the intermediate class with low-miR expression (HR = 2.0, pval = 0.11) to the class with zero lymph nodes, and the high-miR expression (HR = 5.7, pval = 0.003) with the class with five or more lymph nodes.

**Example 7.**

miR-423-5p is a tumor-specific prognostic biomarker for OSCC

To assess whether miR-423-5p has a tumor origin, we compared i) 15 pre- and post-operative saliva samples, coming from our OSCC cohort; and ii) 14 tumors and matched normal tissues, coming from TCGA collection. We observed a significant decrease of miR-423-5p salivary expression after tumor resection (LogFC=2.4, p < 0.001; Figure 5A) and its overexpression in tumor tissues compared to normal controls (LogFC=0.161, p=0.015; Figure 5B). Altogether, these results suggest that miR-423-5p, highly expressed in OSCC tissue and scarcely detected in saliva after tumor resection, may originate from cancer cells. To unravel possible functional roles of miR-423-5p, its putative target genes were included in KEGG pathway annotations to perform the Over-Representation Analysis (361 targets out of 6.229 annotated genes). 52 out of 302 pathways (17.2%) resulted as significantly enriched (adjusted p.val≤0.05,). These included several cancer-related pathways, such as cell signaling (MAPK, Notch), metabolism (N-Glycans biosynthesis) and those related to cell migration. Focal adhesion, cell junctions and gap junctions, in particular, were among the most enriched pathways, suggesting that miR-423-5p might play a role in OSCC progression and metastasis (since most of the biological functions of the target genes are involved in it).

**Methods**

Patient cohort

The present study was performed following the Declaration of Helsinki set of principles and approved by the Research Review Board - the Ethic Committee - of the Spedali Civili, Brescia, Italy (study reference number: NP1545). Written informed consent was obtained from all participants. Saliva samples were collected from consecutive patients with a diagnosis of primary OSCC, which were referred for primary treatment to the Unit of Otorhinolaryngology-Head and Neck Surgery of the Spedali Civili-University of Brescia (Italy), between October 2013 and September 2016 (training set) and between January 2018 and February 2020 (test set). All patients were staged according to the 8th Edition of the AJCC-UICC TNM system. Normal saliva samples were obtained from patients with no oral lesions and matched with OSCC patients regarding smoking and alcohol consumption.

Saliva collection

Patients were requested to refrain from smoking, eating, drinking and oral hygiene procedures for at least 1 h prior to saliva spitting. Unstimulated whole saliva was collected after mouth rinsing with 10 mL of sterile PBS and immediately processed by centrifugation at 2600 g for 15 min at 4°C to remove cellular components, according to a previously published protocol (Momen-Heravi F, Trachtenberg AJ, Kuo WP, Cheng YS. Genomewide study of salivary microRNAs for detection of oral cancer. J Dent Res 2014;93:86s-93s). Saliva supernatant was divided into 250-$\mu$l aliquots and stored at - 80°C until further miRNA extraction.

RNA isolation

Total RNA was extracted from 200 $\mu$l of saliva using miRNeasy Mini kit (Qiagen, Hilden, Germany), with a modified protocol for co-purification of small RNAs from liquid samples (Todeschini P, et al. Circulating miRNA landscape identifies miR-1246 as promising diagnostic biomarker in high-grade serous ovarian carcinoma: A validation across two independent cohorts. Cancer Lett 2017; 388:320-327.).
Ten synthetic spike-in RNA oligos without sequence homology to known human miRNAs were added to samples to control for variations during the preparation of total RNA and subsequent steps, as previously described (Todeschini et al.).

miRNA profiling by microarray

Global profiling of salivary miRNAs was carried out using commercially available G4872A-046064 human miRNA microarray (Agilent Technologies, Santa Clara, CA, USA), customized with probes for the detection of specific RNA oligo spike-in. Fifteen $\mu$l of total RNA were dried by speedvac and resuspended in 2 $\mu$l H2O before being labelled and hybridized on miRNA array, according to miRNA labelling and hybridization kit instructions (Agilent Technologies). Following washing and scanning with laser confocal scanner (G2565BA, Agilent Technologies), miRNA microarrays underwent standard post-hybridization processing and the intensity of fluorescence was calculated by Feature Extraction software version 11 (Agilent Technologies). Raw data are available at GEO database (accession number pending).

miRNA quantification by quantitative real-time PCR

The miRCURY locked-nucleic acid Universal real-time (RT) miRNA PCR system (Exiqon, Qiagen Hilden, Germany), based on universal reverse transcription followed by RT PCR amplification with miRNA-specific primers, was used for first-strand cDNA synthesis and SYBR Green-based amplification.
In total, 4 $\mu$l of total RNA were used as input for reverse transcription following manufacturer's instructions. A total of 4 $\mu$l of a 50X dilution of cDNA were used for subsequent qPCR experiments, run in triplicate using Bio-Rad CFX96 Real-Time system. An inter-run calibration sample was used in all plates to correct for the technical variance between the different runs and to compare results from different plates. The 2-$\Delta\Delta$Cq method was used to analyze raw Cq data following normalization with selected reference miRNAs and results were displayed as relative expression.

miRNA normalization strategy

To accurately quantify miRNA levels and compare them between samples, a proper normalization relative to an endogenous miRNA is mandatory (Schwarzenbach H, et al. Data normalization strategies for microRNA quantification. Clin Chem 2015;61(11):1333-1342.). In the absence of established endogenous miRNAs for reliable expression data normalization, we selected potential candidates according to their use in published studies, carrying out a Medline search using the MeSH terms "salivary miRNA" and "oral cancer" and "real-time PCR".

Validation on external database (TCGA dataset analysis)

Level 3 miRNA sequencing data (RNA-Seq) for the Head and Neck Squamous Cell Carcinoma Tissues (TCGA-HNSC project) were downloaded from GDC Data Portal (NIH) using GDC queries (harmonized dataset). For each sample, mapped reads per millions (RPM) were sum up across unique isoform IDs. Only samples annotated as oral squamous cell carcinomas with matched primary solid tumor (code 01) and normal solid tissue (code 11) were retained. Additional filter criteria were applied to tissue/organ of origin to ensure the comparability of the two collected cohorts. A total of 14 matched tumor-normal samples were selected for further analysis (TCGA code: HD-8635, CV-6961, CV-6959, CV-6939, CV-6933, CV-7238, CV-7235, CV-6936, CV-6934, CV-7103, CV-6956, CV-7255, HD-A6HZ, CV-7438).

Statistical analysis

All statistical analyses were performed using R software (version 3.4).

- Pre-processing and differential expression analysis of microarray data

Salivary raw microarray data comprised 1361 miRNAs repeated 40-times. The expressions of the 40 miRNAs replicates were used as technical replicates and quality control. For pre-processing and normalization steps, we follow the strategy proposed in Todeschini et al. (above). Briefly, we selected only miRNAs with at least 75% of samples -either within patients or healthy controls - with more than 20% good quality replicates (using gisPosandSig Agilent flag). The miRNA replicates within samples were summarized using mean values. The few missing values still present (0.9%) were imputed with k-nearest neighborhood method. Expression data of the remaining 826 miRNAs were normalized using cyclic LOWESS algorithm, in which the ten spikes in oligos were used as stabilizing factors (weight = 10). Empirical Bayes test (limma package) was used for differential expression analysis and Benjamini-Hochberg correction was applied for multiple testing correction.

- RT-qPCR data and equivalence analysis

Normalized RT-qPCR data were obtained as the difference between Cq values of target miRNA and the selected reference ($\Delta$Cq); in case of multiple references, the arithmetic mean computed by the sample was used. Batch effect and age bias were removed from Cq values using the beta coefficients of the linear regression model estimated for each miRNA. Normalized Ct values in different groups were compared, performing one-tail paired sample t-test (OSCC versus healthy; pre- versus post-operative saliva). The equivalence of reference candidates was assessed using both Normfinder approach (Andersen CL, et al. Normalization of real-time quantitative reverse transcription-PCR data: a model-based variance estimation approach to identify genes suited for normalization, applied to bladder and colon cancer data sets. Cancer Res 2004;64(15):5245-5250) (R package, version 5) and the two-one-sided t-tests procedure (Bignotti E, et al. Identification of stably expressed reference small non-coding RNAs for microRNA quantification in high-grade serous ovarian carcinoma tissues. J Cell Mol Med 2016;20(12):2341-2348) (equivalence, R package) with the adoption of the default confidence level (alpha=0.05). To ensure an adequate power of the test the equivalence ranges were chosen depending on data variability following Wellek criteria (Wellek S. Testing statistical hypotheses of equivalence and noninferiority. Chapman and Hall/CRC (2010).

- ROC curves

The Receiver Operating Characteristic (ROC) curves were used to assess diagnostic performances of each miRNA using normalized Ct values. Logistic multivariate model was applied to test the diagnostic performance of the integration of all miRNAs. The numeric value of the area under the ROC curve (AUC) and its confidence interval was calculated with 20 repeated 5-folds cross validation estimates (cvAUC, R package). Each cross-validation fold was stratified by phenotype (OSCC/healthy individual).

- Survival analysis

Disease-free survival (DFS) was defined as the time from the first surgery to the date of the first recurrence or last follow-up. The association of miRNA profiles and DFS was assessed based on the Cox proportional hazard model, with both univariate and multivariate analysis.

- Pathway analysis

miR-423-5p/gene interactions were obtained from the manually curated database DIANA-TarBase (versione 7.0) via web server interfaces. In order to assess whether a certain biological pathway was significantly enriched, we performed an Over-Representation Analysis (ORA) based on the hypergeometric test (Benjamini-Hochberg correction), as proposed by Backes et al. (GeneTrail-advanced gene set enrichment analysis. Nucleic acids Res 2007, 35, W186-W192.). KEGG pathway annotations were retrieved using graphite package (version 1.34.0) (Sales G, et al Graphite - a Bioconductor package to convert pathway topology to gene network. BMC Bioinformatics 2012, Jan 31;13:20).

From the above description and the above-noted examples, the advantage attained by the use of the miR-423-5p miRNA described and obtained according to the present invention are apparent.

The present invention therefore resolves the above-lamented problem of allowing the diagnosisi and the stratification of oral cancer patients, offering at the same time numerous other advantages, including the development of diagnostic methods capable of avoiding invasive techniques and predicting the therapeutic response so to refine not only the diagnostics but above all direct the best therapeutic choice.

**Claims**

1. A method for diagnosing or prognosticating an oral carcinoma, comprising the steps of:

   - determining the levels of expression of the miRNAS miR-423-5p, miR-106b-5p and miR-193b-3p in a sample; and
   - comparing said levels of expression of the miRNAs miR-423-5p, miR-106b-5p and miR-193b-3p in a sample to healthy controls.

2. The method according to claim 1, comprising at least the further step of comparing said levels of expression of the miRNAs miR-423-5p, miR-106b-5p and miR-193b-3p in a sample to the level of expression of the reference miRNAs miR-16-5p, miR-484 and miR-191-5p.

3. The method for diagnosing an oral carcinoma according to any one of claims 1 or 2, wherein said method comprises at least the steps of:

   (a) determining the expression level (Cq) of the target miRNAs miR-423-5p, miR-106b-5p and miR-193b-3p in a sample;
   (b) determining the expression level (Cq) of the reference miRNAs miR-16-5p, miR-484 and miR-191-5p in a sample;
   (c) normalizing the expression level of the target miRNAs to the expression of the reference miRNAs ($\Delta$Cq);
   (d) calculating the *diagnostic score* as linear combinations of normalized expression levels of target miRNAs; and
   e) comparing the diagnostic score with a predetermined cutoff value chosen to exclude oral carcinoma, wherein a diagnostic score above the cutoff value of -0.48426 is indicative of oral carcinoma.

4. The method for making a prognosis of the outcome of an oral carcinoma according to any one of claims 1 or 2, wherein said method comprises at least the steps of:

   (a) determining the expression level (Cq) of the miRNA miR-423-5p in a sample;
   (b) determining the expression level (Cq) of the reference miRNAs miR-16-5p, miR-484 and miR-191-5p in a sample;
   (c) normalizing the expression level of miR-423-5p to the expression of the reference miRNAs ($\Delta$Cq);
   (d) comparing the normalized expression level of miRNA-423-5p with a predetermined cutoff value to predict risk of relapse for oral carcinoma, wherein a normalized expression level above the cutoff value of 0.49 is indicative of an increased risk of oral carcinoma relapse.

**5.** The method according to any one of claims 1 to 4, wherein said sample is a liquid biopsy.

**6.** The method according to any one of claims 1 to 5, wherein sample is a saliva sample.

**7.** The method according to any one of claims 1 to 6, wherein said level of expression determination is carried out by real-time PCR.

**8.** Use of a combination of target miRNAs for diagnosing an oral carcinoma, said combination of target miRNAs consisting of miRNAs miR-423-5p, miR-106b-5p and miR-193b-3p.

**Patentansprüche**

**1.** Verfahren zur Diagnose oder Prognose eines oralen Karzinoms, umfassend die Schritte:

- Bestimmen der Expressionsniveaus der miRNAs miR-423-5p, miR-106b-5p und miR-193b-3p in einer Probe; und
- Vergleichen der Expressionsniveaus der miRNAs miR-423-5p, miR-106b-5p und miR-193b-3p in einer Probe mit gesunden Kontrollen.

**2.** Verfahren nach Anspruch 1, umfassend mindestens den weiteren Schritt des Vergleichens der Expressionsniveaus der miRNAs miR-423-5p, miR-106b-5p und miR-193b-3p in einer Probe mit den Expressionsniveaus der Referenz-miRNAs miR-16-5p, miR-484 und miR-191-5p.

**3.** Verfahren zur Diagnose eines oralen Karzinoms nach einem der Ansprüche 1 oder 2, wobei das Verfahren mindestens die Schritte umfasst:

(a) Bestimmen des Expressionsniveaus (Cq) der Ziel-miRNAs miR-423-5p, miR-106b-5p und miR-193b-3p in einer Probe;
(b) Bestimmen des Expressionsniveaus (Cq) der Referenz-miRNAs miR-16-5p, miR-484 und miR-191-5p in einer Probe;
(c) Normalisieren des Expressionsniveaus der Ziel-miRNAs auf die Expression der Referenz-miRNAs (ΔCq);
(d) Berechnen des *diagnostischen Scores* als lineare Kombinationen von normalisierten Expressionsniveaus von Ziel-miRNAs; und
e) Vergleichen des diagnostischen Scores mit einem vorbestimmten Grenzwert, der so gewählt ist, dass orale Karzinome ausgeschlossen sind, wobei ein diagnostischer Score über dem Grenzwert von -0,48426 auf ein orales Karzinom hinweist.

**4.** Verfahren zur Erstellung einer Verlaufsprognose eines oralen Karzinoms nach einem der Ansprüche 1 oder 2, wobei das Verfahren mindestens die Schritte umfasst:

(a) Bestimmen des Expressionsniveaus (Cq) der miRNA miR-423-5p in einer Probe;
(b) Bestimmen des Expressionsniveaus (Cq) der Referenz-miRNAs miR-16-5p, miR-484 und miR-191-5p in einer Probe;
(c) Normalisieren des Expressionsniveaus von miR-423-5p auf die Expression der Referenz-miRNAs (ΔCq);
(d) Vergleichen des normalisierten Expressionsniveaus von miRNA-423-5p mit einem vorbestimmten Grenzwert, um ein Rückfallrisiko für ein orales Karzinom vorherzusagen, wobei das normalisierte Expressionsniveau über dem Grenzwert von 0,49 auf ein erhöhtes Rückfallrisiko für ein orales Karzinom hinweist.

**5.** Verfahren nach einem der Ansprüche 1 bis 4, wobei die Probe eine Flüssigbiopsie ist.

**6.** Verfahren nach einem der Ansprüche 1 bis 5, wobei die Probe eine Speichelprobe ist.

**7.** Verfahren nach einem der Ansprüche 1 bis 6, wobei die Bestimmung des Expressionsniveaus durch Echtzeit-PCR durchgeführt wird.

**8.** Verwendung einer Kombination von Ziel-miRNAs zur Diagnose eines oralen Karzinoms, wobei die Kombination von Ziel-miRNAs aus den miRNAs miR-423-5p, miR-106b-5p und miR-193b-3p besteht.

**Revendications**

1. Procédé de diagnostic ou de pronostic d'un carcinome buccal, comprenant les étapes suivantes :

   - déterminer les niveaux d'expression des miRNAS miR-423-5p, miR-106b-5p et miR-193b-3p dans un échantillon ; et
   - comparer lesdits niveaux d'expression des miRNAs miR-423-5p, miR-106b-5p et miR-193b-3p dans un échantillon à des sujets témoins.

2. Procédé selon la revendication 1, comprenant au moins l'étape supplémentaire consistant à comparer lesdits niveaux d'expression des miRNAs miR-423-5p, miR-106b-5p et miR-193b-3p dans un échantillon au niveau d'expression des miRNAs de référence miR-16-5p, miR-484 et miR-191-5p.

3. Procédé de diagnostic d'un carcinome buccal selon l'une quelconque des revendications 1 ou 2, dans lequel ledit procédé comprend au moins les étapes consistant à :

   (a) déterminer le niveau d'expression (Cq) des miRNAs cibles miR-423-5p, miR-106b-5p et miR-193b-3p dans un échantillon ;
   (b) déterminer le niveau d'expression (Cq) des miRNAs de référence miR-16-5p, miR-484 et miR-191-5p dans un échantillon ;
   (c) normaliser le niveau d'expression des miRNAs cibles à l'expression des miRNAs de référence ($\Delta$Cq) ;
   (d) calculer le *résultat de diagnostic* comme des combinaisons linéaires de niveaux d'expression normalisés de miRNAs cibles ; et
   e) comparer le résultat de diagnostic avec une valeur de coupure prédéterminée choisie pour exclure le carcinome buccal, dans lequel un résultat de diagnostic supérieur à la valeur de coupure de -0,48426 est indicatif du carcinome buccal.

4. Procédé pour établir un pronostic de l'évolution d'un carcinome buccal selon l'une quelconque des revendications 1 ou 2, dans lequel ledit procédé comprend au moins les étapes consistant à :

   (a) déterminer le niveau d'expression (Cq) du miRNA miR-423-5p dans un échantillon ;
   (b) déterminer le niveau d'expression (Cq) des miRNAs de référence miR-16-5p, miR-484 et miR-191-5p dans un échantillon ;
   (c) normaliser le niveau d'expression de miR-423-5p à l'expression des miRNAs de référence ($\Delta$Cq) ;
   (d) comparer le niveau d'expression normalisé du miRNA-423-5p avec une valeur de coupure prédéterminée pour prédire le risque de rechute pour le carcinome buccal, dans lequel un niveau d'expression normalisé supérieur à la valeur de coupure de 0,49 est indicatif d'un risque accru de rechute du carcinome buccal.

5. Procédé selon l'une quelconque des revendications 1 à 4, dans lequel ledit échantillon est une biopsie liquide.

6. Procédé selon l'une quelconque des revendications 1 à 5, dans lequel l'échantillon est un échantillon de salive.

7. Procédé selon l'une quelconque des revendications 1 à 6, dans lequel ledit niveau de détermination d'expression est effectué par PCR en temps réel.

8. Utilisation d'une combinaison de miRNAs cibles pour diagnostiquer un carcinome buccal, ladite combinaison de miRNAs cibles consistant en les miRNAs miR-423-5p, miR-106b-5p et miR-193b-3p.

Figure 1.

**Figure 2.**

**Figure 3.**

**A**

miR-106-5p (AUC=0.81)
miR-423-5p (AUC=0.85)
miR-193b-3p (AUC=0.75)
Training - Logit (AUC=0.98)

Sensitivity

1-Specificity

**B**

Merged - Logit (AUC=0.92)
True positive proportion: 0.854
True negative proportion: 0.149

Sensitivity

1-Specificity

Figure 4.

**Figure 5.**

A

B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020068673 A

**Non-patent literature cited in the description**

- **CHANG YI-AN et al.** *INTERNATIONAL JOURNAL OF MOLECULAR SCIENCES*, 07 March 2018, vol. 19 (3)
- **MATTAVELLI D et al.** Prognostic nomograms in oral squamous cell carcinoma: the negative impact of low neutrophil to lymphocyte ratio. *Front Oncol*, 2019, vol. 9, 339
- **BIANCHI F et al.** A serum circulating miRNA diagnostic test to identify asymptomatic high-risk individuals with early stage lung cancer. *EMBO Mol Med*, 2011, vol. 3 (8), 495-503
- **BUSTIN SA et al.** The MIQE guidelines: minimum information for publication of quantitative real-time PCR experiments. *Clin Chem*, 2009, vol. 55 (4), 611-622
- Metastatic lymph node burden and survival in oral cavity cancer. *J Clin Oncol*, 2017, vol. 35 (31), 3601-3609
- **MOMEN-HERAVI F ; TRACHTENBERG AJ ; KUO WP ; CHENG YS**. Genomewide study of salivary microRNAs for detection of oral cancer. *J Dent Res*, 2014, vol. 93, 86s-93s
- **TODESCHINI P et al.** Circulating miRNA landscape identifies miR-1246 as promising diagnostic biomarker in high-grade serous ovarian carcinoma: A validation across two independent cohorts.. *Cancer Lett*, 2017, vol. 388, 320-327
- **SCHWARZENBACH H et al.** Data normalization strategies for microRNA quantification.. *Clin Chem*, 2015, vol. 61 (11), 1333-1342
- **ANDERSEN CL et al.** Normalization of real-time quantitative reverse transcription-PCR data: a model-based variance estimation approach to identify genes suited for normalization, applied to bladder and colon cancer data sets. *Cancer Res*, 2004, vol. 64 (15), 5245-5250
- **BIGNOTTI E et al.** Identification of stably expressed reference small non-coding RNAs for microRNA quantification in high-grade serous ovarian carcinoma tissues. *J Cell Mol Med*, 2016, vol. 20 (12), 2341-2348
- **WELLEK S**. Testing statistical hypotheses of equivalence and noninferiority. Chapman and Hall/CRC, 2010
- **BACKES et al.** GeneTrail-advanced gene set enrichment analysis. *Nucleic acids Res*, 2007, vol. 35, W186-W192
- **SALES G et al.** Graphite - a Bioconductor package to convert pathway topology to gene network. *BMC Bioinformatics*, 31 January 2012, vol. 13, 20